(19) Europäisches Patentamt
European Patent Office
Office européen des brevets

(11) **EP 2 035 141 B1**

(12) **FASCICULE DE BREVET EUROPEEN**

(45) Date de publication et mention
de la délivrance du brevet:
**31.01.2018   Bulletin 2018/05**

(21) Numéro de dépôt: **07765975.3**

(22) Date de dépôt: **31.05.2007**

(51) Int Cl.:
*B01J 35/00* (2006.01)   *B01J 23/46* (2006.01)
*B01J 35/10* (2006.01)   *B01J 37/08* (2006.01)
*B01J 37/18* (2006.01)   *B22F 1/00* (2006.01)
*B22F 9/26* (2006.01)   *B22F 9/30* (2006.01)
*G01N 33/00* (2006.01)   *B82Y 40/00* (2011.01)
*B82Y 30/00* (2011.01)

(86) Numéro de dépôt international:
**PCT/FR2007/000905**

(87) Numéro de publication internationale:
**WO 2007/138197 (06.12.2007 Gazette 2007/49)**

(54) **PREPARATION DE NANOMATERIAUX HYBRIDES ET FILTRES POUR CAPTEURS DE GAZ**

HERSTELLUNG VON HYBRIDNANOMATERIALIEN UND FILTERN FÜR GASFALLEN

PREPARATION OF HYBRID NANOMATERIALS AND FILTERS FOR GAS TRAPS

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité:  **01.06.2006   FR 0604896**

(43) Date de publication de la demande:
**18.03.2009   Bulletin 2009/12**

(73) Titulaires:
• **Centre National de la Recherche Scientifique (C.N.R.S.)**
**75016 Paris (FR)**
• **Universite de Montpellier II**
**34095 Montpellier Cedex 5 (FR)**

(72) Inventeurs:
• **PHILIPPOT, Karine**
**31450 Montbrun Lauragais (FR)**
• **MAISONNAT, André**
**31120 Roquettes (FR)**
• **CHAUDRET, Bruno**
**31320 Vigoulet Auzil (FR)**
• **JANSAT, Susanna**
**08304 Mataro (Barcelonne) (ES)**
• **GARCIA-ANTON, Jordi**
**08201 Sabadell (ES)**
• **TURPIN, Florence**
**69780 Mions (FR)**
• **MATSURA, Victor**
**199178 Saint-Petersbourg (RU)**
• **REYE, Catherine**
**34080 Montpellier (FR)**
• **GUARI, Yannick**
**34730 Prades-le-Lez (FR)**
• **CORRIU, Robert**
**34090 Montpellier (FR)**

(74) Mandataire: **Lavoix**
**2, place d'Estienne d'Orves**
**75441 Paris Cedex 09 (FR)**

(56) Documents cités:
**WO-A-99/01766**

• **CABOT A ET AL: "Mesoporous catalytic filters for semiconductor gas sensors", THIN SOLID FILMS, ELSEVIER-SEQUOIA S.A. LAUSANNE, CH, vol. 436, no. 1, 22 juillet 2003 (2003-07-22), pages 64-69, XP004431392, ISSN: 0040-6090, DOI: 10.1016/S0040-6090(03)00510-8**
• **STANGER K J ET AL: "Catalytic oxidation of a thioether and dibenzothiophenes using an oxorhenium(V) dithiolate complex tethered on silica", JOURNAL OF MOLECULAR CATALYSIS A: CHEMICAL, ELSEVIER, AMSTERDAM, NL, vol. 243, no. 2, 16 janvier 2006 (2006-01-16), pages 158-169, XP028015593, ISSN: 1381-1169, DOI: 10.1016/J.MOLCATA.2005.07.019 [extrait le 2006-01-16]**

- WILLIAM H. PIRKLE ET AL: "A chiral stationary phase which affords unusually high levels of enantioselectivity", CHIRALITY., vol. 3, no. 3, 1 janvier 1991 (1991-01-01) , pages 183-187, XP055262815, US ISSN: 0899-0042, DOI: 10.1002/chir.530030308
- GEROLAMO BUDRONI ET AL: "Gold-Organic-Inorganic High-Surface-Area Materials as Precursors of Highly Active Catalysts", ANGEWANDTE CHEMIE INTERNATIONAL EDITION, vol. 45, no. 20, 12 mai 2006 (2006-05-12), pages 3328-3331, XP055262861, DE ISSN: 1433-7851, DOI: 10.1002/anie.200600552
- APRILE C ET AL: "Synthesis and catalytic activity of periodic mesoporous materials incorporating gold nanoparticles", JOURNAL OF MATERIALS CHEMISTRY, ROYAL SOCIETY OF CHEMISTRY, GB, vol. 15, no. 41, 7 novembre 2005 (2005-11-07), pages 4408-4413, XP002528288, ISSN: 0959-9428, DOI: 10.1039/B507418E [extrait le 2005-08-23]
- LEE B ET AL: "Preparation of bicontinuous mesoporous silica and organosilica materials containing gold nanoparticles by co-synthesis method" MICROPOROUS AND MESOPOROUS MATERIALS, ELSEVIER SCIENCE PUBLISHING, NEW YORK, US, vol. 70, no. 1-3, 21 mai 2004 (2004-05-21), pages 71-80, XP004506225 ISSN: 1387-1811
- CARPENTER JOSEPH P ET AL: "Nanocomposites from molecularly doped silica xerogels: an overview" INT SAMPE TECH CONF; INTERNATIONAL SAMPE TECHNICAL CONFERENCE 1995 SAMPE, COVINA, CA, USA, vol. 27, 1995, pages 549-559, XP008073396
- HULEA V ET AL: "Synthesis of well-dispersed ruthenium nanoparticles inside mesostructured porous silica under mild conditions" MICROPOROUS AND MESOPOROUS MATERIALS, ELSEVIER SCIENCE PUBLISHING, NEW YORK, US, vol. 79, no. 1-3, 1 avril 2005 (2005-04-01), pages 185-194, XP004784525 ISSN: 1387-1811
- PELZER K ET AL: "New Ru nanoparticles stabilized by organosilane fragments" CHEM. MATER.; CHEMISTRY OF MATERIALS NOV 30 2004, vol. 16, no. 24, 30 novembre 2004 (2004-11-30), pages 4937-4941, XP002414303
- CABOT A ET AL: "Mesoporous catalytic filters for semiconductor gas sensors", THIN SOLID FILMS, ELSEVIER-SEQUOIA S.A. LAUSANNE, CH, vol. 436, no. 1, 22 July 2003 (2003-07-22) , pages 64-69, XP004431392, ISSN: 0040-6090, DOI: 10.1016/S0040-6090(03)00510-8
- STANGER K J ET AL: "Catalytic oxidation of a thioether and dibenzothiophenes using an oxorhenium(V) dithiolate complex tethered on silica", JOURNAL OF MOLECULAR CATALYSIS A: CHEMICAL, ELSEVIER, AMSTERDAM, NL, vol. 243, no. 2, 16 January 2006 (2006-01-16), pages 158-169, XP028015593, ISSN: 1381-1169, DOI: 10.1016/J.MOLCATA.2005.07.019 [retrieved on 2006-01-16]
- WILLIAM H. PIRKLE ET AL: "A chiral stationary phase which affords unusually high levels of enantioselectivity", CHIRALITY., vol. 3, no. 3, 1 January 1991 (1991-01-01) , pages 183-187, XP055262815, US ISSN: 0899-0042, DOI: 10.1002/chir.530030308
- GEROLAMO BUDRONI ET AL: "Gold-Organic-Inorganic High-Surface-Area Materials as Precursors of Highly Active Catalysts", ANGEWANDTE CHEMIE INTERNATIONAL EDITION, vol. 45, no. 20, 12 May 2006 (2006-05-12), pages 3328-3331, XP055262861, DE ISSN: 1433-7851, DOI: 10.1002/anie.200600552
- APRILE C ET AL: "Synthesis and catalytic activity of periodic mesoporous materials incorporating gold nanoparticles", JOURNAL OF MATERIALS CHEMISTRY, ROYAL SOCIETY OF CHEMISTRY, GB, vol. 15, no. 41, 7 November 2005 (2005-11-07), pages 4408-4413, XP002528288, ISSN: 0959-9428, DOI: 10.1039/B507418E [retrieved on 2005-08-23]

**Description**

La présente invention concerne le domaine de la catalyse hétérogène et, plus précisément, celui des filtres catalytiques, notamment pour les capteurs de gaz.

Dans ce domaine, les dispositifs aujourd'hui commercialisés sont équipés de filtres dits "passifs". Le matériau généralement utilisé pour cette opération est de type charbon actif (Schweizer-Berberich et al., Sens. Actuators, B, 2000, 66, 34). Plusieurs inconvénients sont attachés à l'usage de tels matériaux. Ils sont notamment peu sélectifs et ont une durée de vie limitée en raison de la saturation.

La conception de matériaux pour filtres catalytiques est étudiée depuis quelques années (Arbiol et al., Applied Phys. Lett., 2002, 81, 3449). Il s'agit de matériaux composites comprenant un catalyseur dispersé dans une matrice solide qui agissent par transformation sélective des gaz interférents. Ces filtres catalytiques ne sont donc pas saturables. Par ailleurs, l'efficacité du filtre dépend de la sélectivité du catalyseur choisi. La réalisation de tels matériaux est cependant délicate dans la mesure où le contrôle de la composition et de la morphologie de ces matériaux à l'échelle nanométrique est primordial pour une meilleure efficacité et pour des résultats reproductibles. De plus, la mise en oeuvre de tels matériaux doit être compatible avec les procédés de la microélectronique, notamment l'intégration à la surface de la couche sensible du capteur de gaz.

Lee et al., Microporous and mesoporous materials, vol. 70, 1-3, 2004, 71-80, décrit un procédé de préparation d'un nanomatériau comprenant des nanoparticules métalliques.

Carpenter et al., Microporous and mesoporous materials, vol. 79, 1-3, 2005, 185-194, décrit des procédés de préparation de nanomatériaux.

Hurea et al., Microporous and mesoporous materials, vol. 79, 1-3, 2005, 185-194, décrit la préparation de nanoparticules.

Il est donc particulièrement désirable de mettre à disposition de nouveaux matériaux et un procédé de préparation amélioré permettant la réalisation de filtres catalytiques non saturables, sélectifs, de composition homogène et de morphologie contrôlée.

Les présents inventeurs ont découvert un nouveau mode de synthèse conduisant de façon reproductible à un nanomatériau hybride dont la composition et la morphologie sont parfaitement contrôlées. Ledit procédé permet la dispersion du catalyseur de façon homogène à l'intérieur de la matrice. Le nouveau matériau hybride ainsi préparé peut être aisément mis en oeuvre à la surface de la couche sensible semi-conductrice d'un capteur de gaz, par les techniques habituelles, par exemple au moyen d'un micro-injecteur.

Selon un premier objet, la présente invention concerne donc un procédé de préparation d'un nanomatériau hybride contenant des nanoparticules métalliques, éventuellement oxydées, comprenant :

1) la formation de nanoparticules dudit métal, à partir d'un précurseur dudit métal, en présence d'un ligand (L) choisi parmi $(PhCH_2)_2N(CH_2)_{11}O(CH_2)_3Si(OEt)_3$ ou $H_2N(CH_2)_{11}O(CH_2)_3Si(OEt)_3$, sous pression d'hydrogène ;

2) la polymérisation du ligand (L) au sein d'un matériau solide, et

3) la calcination du matériau obtenu.

La polymérisation peut être éventuellement suivie d'une étape de lavage ou séchage, avant l'étape de calcination éventuelle.

Le lavage peut être réalisé au moyen de tout solvant dans lequel le matériau est insoluble. On peut notamment citer le pentane, l'hexane, les éthers de pétrole et le tétrahydrofurane.

Le séchage peut être avantageusement réalisé à l'air.

A titre de métal, on peut utiliser tout métal de transition, notamment les métaux des groupes VIII de la classification périodique, tels que Pd, Pt, Ru ou encore tout autre métal pour lequel des complexes métalliques proches du degré d'oxydation 0 sont disponibles, par exemple Rh, Co, Ni, Fe, Au, Cu; on préfère notamment le ruthénium.

Le précurseur dudit métal peut notamment être un complexe du métal considéré. De façon avantageuse, ledit complexe peut être disponible commercialement ou être préparé selon les méthodes connues. Dans le cas du ruthénium, on préfère notamment le complexe Ru(COD)(COT) où COD=1,5-cyclooctadiène et COT=1,3,5-cyclooctatriène.

A titre de ligand, on décrit les ligands stabilisants bifonctionnels, comportant une fonction polymérisable de formule (L).

Dans la formule (L)

Y-X-G          (L)

G représente un groupe hydrolysable, polycondensable tel que $-Si(OR'')_3$ dans lequel R'' est un groupe alkyle en C1-C20, de préférence C1-C10, tel que -Si(OEt)$_3$,-Si(OMe)$_3$ ou -Si(O$^i$Pr)$_3$.

Y représente un groupe choisi parmi les amines -NRR', les thiols, les phosphines ou les groupements phosphonate, phosphate ou carboxylate,

avec R et R', identiques ou différents, choisis parmi un atome d'hydrogène, un groupe alkyle, aralkyle ou aryle dans lequel alkyle est un groupe C$_1$-C$_{20}$ alkyle, de préférence C$_8$-C$_{16}$ et aryle un groupe C$_6$-C$_{12}$ aryle, de préférence C$_6$,

X est une chaîne hydrocarbonée, linéaire ou ramifiée, comprenant au moins 4 maillons, comprenant de 1 à 40 atomes de carbone, et pouvant contenir éventuellement un ou plusieurs hétéroatome(s) tels que O ou N ; plus préférentiellement, X contient de 10 à 40 atomes de carbone et plus préférentiellement entre 10 et 20 atomes de carbone.

Plus préférentiellement :

G représente -Si(OEt)$_3$.

Y représente -NRR' avec R et R' choisis parmi un atome d'hydrogène ou un groupe méthyle ou benzyle.

X est une chaîne hydrocarbonée linéaire ou ramifiée, comprenant au moins 4 maillons, comprenant de 1 à 20 atomes de carbone, et pouvant contenir éventuellement un atome d'oxygène.

Selon l'invention, le ligand est choisi parmi (PhCH$_2$)$_2$N(CH$_2$)$_{11}$O(CH$_2$)$_3$Si(OEt)$_3$ ou H$_2$N(CH$_2$)$_{11}$O(CH$_2$)$_3$Si(OEt)$_3$

Généralement, l'étape 1) est réalisée à une pression comprise entre 2 et 10 bars ; plus préférentiellement, à 3 bars.

Généralement, cette réaction est conduite dans un autoclave de type Fischer-Porter ou tout autre réacteur équivalent.

Généralement, on opère dans un milieu solvant organique, le choix du solvant dépendant de la solubilité du ligand et du métal ou du précurseur métallique. On préfère notamment le THF, le méthanol, le toluène ou leurs mélanges.

La réaction 1) est conduite pendant une période suffisante pour obtenir un degré d'avancement satisfaisant dans la réaction ; généralement, la réaction est conduite entre 5 et 48 heures ; plus préférentiellement, pendant environ 20 heures.

La polymérisation 2) est généralement réalisée en présence de tétraalkoxysilane Si(OEt)$_4$, Si(OMe)$_4$ ou Si(O$^i$Pr)$_4$ par évaporation du solvant ou l'addition d'un catalyseur nucléophile et d'eau. Le catalyseur nucléophile peut être choisi parmi le fluorure de tétrabutylammonium ou le fluorure de sodium ; préférentiellement, le catalyseur est ajouté dans un rapport de 5 % à 20 % molaire par rapport au ligand ; de l'eau, préférentiellement en quantité stoechiométrique, est ajoutée.

L'étape de polymérisation consiste en la formation de liaisons -Si-O-, formant ainsi une structure solide, dans laquelle les particules métalliques sont piégées.

Généralement on opère en présence de 0,5 à 2 équivalents de Si(OEt)$_4$, la quantité de Si(OEt)$_4$ permettant de moduler la dispersion des nanoparticules de métal dans la structure.

Avantageusement, Si(OEt)$_4$ peut être présent dans le mélange réactionnel dès l'étape 1), la polymérisation n'étant initiée que lors de l'évaporation du solvant ou de l'ajout du catalyseur.

L'étape de calcination 3) est réalisée pour éliminer toute la partie organique. Au cours de cette étape, la structure du matériau évolue pour adopter une texture mésoporeuse.

Eventuellement cette étape de calcination peut être réalisée sous air pour obtenir des particules d'oxydes dudit métal. La calcination sous air peut être réalisée par chauffage progressif de l'échantillon de la température ambiante à une température comprise entre 400 et 700°C, pendant une durée comprise entre 6 et 9 heures. Généralement, plus la température maximale est faible et plus la durée de calcination est longue. De préférence, la température maximale est inférieure à 500°C, préférentiellement 400°C, pendant une durée comprise entre 3 et 10 heures. De façon préférentielle, le chauffage est réalisé progressivement avec une pente comprise entre 1° et 10°C par minute, préférentiellement environ 2°C par minute, suivi d'un palier à la température maximale atteinte de 400°C pendant environ 5 heures.

A l'issue de l'étape 2), on peut éventuellement réaliser les étapes de lavage et/ou séchage du matériau obtenu, avant la mise en oeuvre de l'étape 3). Généralement, le lavage est réalisé au moyen de pentane. Le séchage peut être réalisé par toute méthode connue, notamment le séchage à l'air ou dans une étuve.

A l'issue du procédé selon l'invention, on obtient un matériau comprenant des particules de métal éventuellement oxydées, immobilisées de façon stable et homogène dans une structure de silice solide. Les particules sont de taille moyenne contrôlée et homogène, comprise entre 2 et 5 nm, préférentiellement environ 2,5 nm. Généralement, la surface spécifique, contrôlée, est comprise entre 400 et 750 m$^2$/g, plus préférentiellement entre 450 et 500 m$^2$/g.

Le nanomatériau hybride constitué de nanoparticules métalliques éventuellement oxydées obtenu selon le procédé de l'invention peut être caractérisé par les propriétés suivantes :

1) une structure organisée caractérisée par la présence d'un pic de diffraction aux petits angles montrant l'existence de la répétitivité au sein du matériau d'un motif dont la dimension est voisine de la taille des nanoparticules présentes, soit 2 nm,

2) une surface spécifique définie, généralement comprise entre 400 et 750 m$^2$/g, plus préférentiellement entre 450 et 500 m$^2$/g,

3) un diamètre de pores défini compris entre 2 et 3 nm,

4) une structure stable sur une gamme importante de températures, allant de l'ambiante jusqu'à 700°C.

On décrit également le nanomatériau hybride constitué de nanoparticules d'oxyde métallique susceptible d'être obtenu par le procédé selon l'invention.

Selon un autre objet, la présente invention concerne également la préparation d'un filtre pour capteur comprenant les étapes de :

1) préparation du nanomatériau hybride selon le procédé de l'invention ;

2) dispersion du nanomatériau hybride selon l'invention dans un solvant organique ; et

3) dépôt de la suspension sur la surface de la couche sensible d'un capteur.

Généralement, la concentration du nanomatériau hybride selon l'invention est comprise entre 1 et 5 mg pour 0,3 ml de solvant, préférentiellement environ 3mg/0,3ml. Le solvant est tel qu'il permet un dépôt de bonne qualité lors de son évaporation lente. Ledit solvant peut être choisi parmi l'anisole, le toluène ou le THF.

La couche sensible sur laquelle est déposée le nanomatériau hybride est généralement constituée d'oxydes métalliques nanostructurés, tel que $SnO_2$, éventuellement dopé au palladium ou platine, ou d'autres oxydes tels que $ZnO$, $In_2O_3$, $WO_3$.

Le capteur peut être choisi parmi les capteurs habituellement utilisés et notamment les capteurs à plateformes silicium micro-usinées.

Le dépôt est réalisé par tout moyen, notamment par dépôt d'une suspension du nanomatériau hybride par un micro-injecteur et évaporation du solvant.

On décrit également un filtre pour capteur comprenant un nanomatériau hybride selon l'invention déposé sur la couche sensible d'un capteur.

Lesdits nanomatériaux sont particulièrement utiles quand ils sont mis en oeuvre à la surface de couches sensibles semiconductrices de capteurs de gaz. Ils agissent alors comme filtres catalytiques et permettent d'augmenter la sélectivité à un gaz donné. Ils permettent donc d'améliorer la détection sélective dudit gaz, notamment dans un environnement pollué.

Les filtres peuvent en effet effectuer une identification sélective de gaz, notamment par oxydation ou réduction sélective, notamment par ajustement des conditions de fonctionnement, telles que la température.

Les filtres ici décrits sont particulièrement avantageux en ce qu'ils peuvent fonctionner en mode isotherme ou pulsé.

Le contrôle de l'homogénéité de la répartition du métal dans la structure mésoporeuse permet de contrôler la reproductibilité des propriétés du filtre. Les filtres selon l'invention sont particulièrement utiles dans le domaine de la catalyse hétérogène et plus spécifiquement en tant que filtres catalytiques pour capteurs de gaz, notamment dans des applications grand public (détection de fuite de gaz, de mauvaise combustion, contrôle de la qualité de l'air, etc.) ou industrielle (automatisation de procédés, détection d'incendies...).

Les ligands de formule (L) discutés ci-dessus convenant particulièrement à la préparation du nanomatériau hybride sont décrits également. Ils répondent à la formule (L) :

$$Y\text{-}X\text{-}Si(OEt)_3 \qquad (L)$$

dans laquelle :

Y représente un groupe choisi parmi les amines NRR', les thiols, les phosphines, ou les groupements phosphonate, phosphate ou carboxylate

avec R et R', identiques ou différents, choisis parmi un atome d'hydrogène, un groupe alkyle, aryle ou aralkyle, dans lequel alkyle est un groupe $C_1$-$C_{20}$ alkyle, de préférence $C_8$-$C_{16}$ et aryle un groupe $C_6$-$C_{12}$ aryle, de préférence $C_6$,

X est une chaîne hydrocarbonée, linéaire ou ramifiée, comprenant de 1 à 40 atomes de carbone, et pouvant contenir éventuellement un ou plusieurs hétéroatome(s) tels que O ou N ; plus préférentiellement, X contient de 1 à 20 atomes de carbone et plus préférentiellement entre 10 et 20 atomes de carbone,

A l'exception du composé $Me_2N(CH_2)_4Si(OEt)_3$

On peut notamment citer le ligand $(PhCH_2)_2N(CH_2)_{11}O(CH_2)_3Si(OEt)_3$ ou $H_2N(CH_2)_{11}O(CH_2)_3Si(OEt)_3$.

On décrit également le procédé de préparation dudit ligand (L) au moyen des composés de formule (I) et (II) :

$$Y-X' \qquad (I)$$

et

$$H-Si(OEt)_3 \qquad (II)$$

dans lesquelles Y est défini tel qu'en formule (L) et X' représente un groupe précurseur de X.

On entend par groupe précurseur X' tout groupe permettant d'obtenir le groupe correspondant X désiré par toute réaction de couplage, protection/ déprotection, addition, substitution, etc. De préférence, X' correspond à X par élimination d'un atome d'hydrogène.

Ladite réaction est mise en oeuvre en présence d'un réactif tel que le $[Pt_2(divinyltetraméthyldisiloxane)_3]$ dans un excès de $HSi(OEt)_3$.

La figure 1 représente l'image par microscopie électronique à transmission du nanomatériau hybride selon l'invention mettant en évidence une répartition homogène de nanoparticules d'oxyde de ruthénium dont la taille moyenne est de 2,5 nm, avec une dispersion étroite de tailles autour de cette moyenne.

La figure 2 est une représentation schématique de l'utilisation du nanomatériau hybride pour la préparation d'un filtre catalytique sur plateforme de silicium.

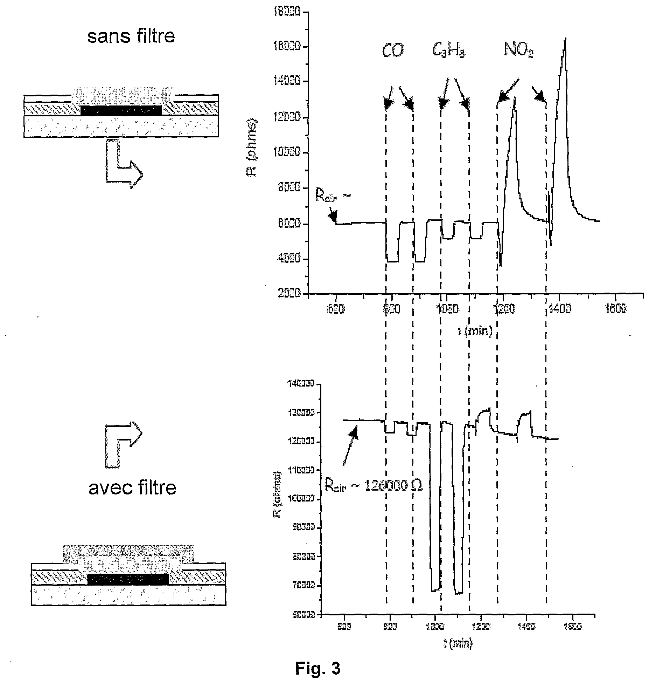

La figure 3 représente les variations de la résistance de la couche sensible des capteurs comprenant ou non le filtre selon l'invention, exposé à des atmosphères constituées de 200 ppm de monoxyde de carbone, 150 ppm de propane, puis 1,8 ppm de $NO_2$ dans l'air humide (50 %).

Les exemples suivants sont donnés à titre illustratif et non limitatif de la présente invention.

Exemple 1

Synthèse du nanomatériau hybride

Des nanoparticules de ruthénium sont synthétisées par décomposition du complexe de ruthénium Ru(COD)(COT), avec COD=1,5-cyclooctadiène et COT=1,3,5-cyclooctatriène, sous pression d'hydrogène en présence du ligand stabilisant bifonctionnel $(PhCH_2)_2N(CH_2)_{11}O(CH_2)_3Si(OEt)_3$ selon le procédé suivant :

100 mg (0,31 mmol) de Ru(COD)(COT), 62 mg de $Si(OEt)_4$ et 172 mg (0,31 mmol) du ligand L dans 10 mL de tétrahydrofurane (THF) sont placés sous trois bars d'hydrogène pendant 20 heures dans un réacteur de type Fischer-Porter. Le matériau peut être obtenu directement par évaporation du solvant ou par ajout de fluorure de tétrabutylammonium (10 % molaire par rapport au ligand) et la quantité stoechiométrique d'eau.

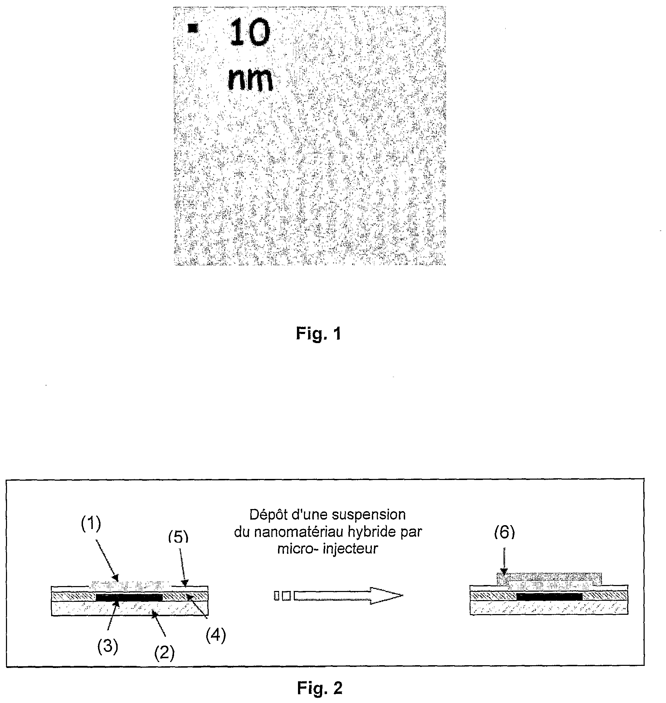

Quelle que soit la méthode d'obtention du solide, celui-ci est ensuite lavé au pentane, puis séché sous vide et enfin calciné sous air. L'étape de calcination est réalisée sous air par une montée en température de 20° à 400°C avec une pente de 2°C par minute, suivie d'un palier à 400°C pendant 5 heures. Le matériau hybride ainsi obtenu est caractérisé par une grande surface spécifique, de l'ordre de 460 $m^2$/g, mesurée par BET. Sa caractérisation par microscopie électronique à transmission telle qu'illustrée à la figure 1 met en évidence une répartition homogène de nanoparticules d'oxyde de ruthénium dont la taille moyenne est de 2,5 nm, avec une dispersion étroite de tailles autour de cette moyenne.

Exemple 2

Mise en oeuvre du nanomatériau hybride selon l'invention en tant que filtre catalytique sur plateforme silicium

Après calcination sous air, le matériau solide obtenu est mis en suspension dans un solvant tel que l'anisole. Une goutte de cette suspension est déposée sur la couche sensible (1) d'un microcapteur de gaz. Le capteur de gaz est constitué d'une isolation thermique (membrane diélectrique) (2), recouverte partiellement d'une résistance chauffante (silicium polycristallin) (3), lesdites isolation thermique et résistance chauffante étant recouvertes d'une isolation électrique (4), telle que $SiO_2$. Sur la surface de l'isolation électrique (4), sont positionnées des électrodes (5) et la couche sensible (1). La suspension est déposée sur la couche sensible (1) pour former le filtre catalytique (6). L'évaporation du solvant laisse une couche épaisse du nanomatériau hybride, répartie sur toute la surface de la couche sensible, qui agit ensuite comme filtre catalytique.

La préparation du filtre catalytique est représentée de façon schématique à la figure 2.

Exemple 3

Propriétés des filtres catalytiques selon l'invention

Les propriétés des filtres catalytiques selon l'invention ont été testées en étudiant la résistance de la couche sensible des capteurs à gaz, exposés à des atmosphères constituées de 200 ppm de CO, 150 ppm de propane, puis 1,8 ppm de $NO_2$ dans l'air humide à 50 %, dans les conditions expérimentales suivantes :

$V_h$ = 3,2 volts
$R_h$ = 50 %
Flux de gaz : 1000 mL/minute

La sensibilité du gaz est calculée par la formule suivante :

$$S_{gaz} = (R_{gaz} - R_{air})/R_{air}.$$

$$S_{co200} - 0,37 \ ; \ S_{C3H8150} = -0,16 \ ; \ S_{NO2\ 1,8} = + 1,5.$$

On remarque une augmentation importante de la sensibilité au propane (triplée) quand la couche sensible est surmontée du filtre catalytique ; la sensibilité au monoxyde de carbone est diminuée d'un facteur 12 tandis que la sensibilité au $NO_2$ est diminuée d'un facteur 30.

Le système couche sensible $SnO_2$ particulaire combiné au matériau hybride $SiO_2/RuO_2$ permet donc la détection d'un hydrocarbure de type propane dans un environnement pollué par CO et $NO_2$.

**Revendications**

1.  Procédé de préparation d'un nanomatériau hybride contenant des nanoparticules métalliques, éventuellement oxydées, comprenant :

    1) la formation de nanoparticules dudit métal, à partir d'un précurseur dudit métal, en présence d'un ligand (L) choisi parmi $(PhCH_2)_2N(CH_2)_{11}O(CH_2)_3Si(OEt)_3$ ou $H_2N(CH_2)_{11}O(CH_2)_3Si(OEt)_3$, sous pression d'hydrogène ;
    2) la polymérisation du ligand (L) au sein d'un matériau solide, et
    3) la calcination du matériau obtenu.

2.  Procédé selon la revendication 1 tel que la polymérisation est suivie d'une étape de lavage ou séchage, avant l'étape de calcination.

3.  Procédé selon la revendication 1 ou 2 tel que le métal est choisi parmi Pd, Pt, Ru, Rh, Co, Ni, Fe, Au, Cu.

4.  Procédé selon l'une quelconque des revendications précédentes tel que le précurseur métallique est Ru(COD)(COT) où COD=1,5-cyclooctadiène et COT=1,3,5-cyclooctatriène.

5.  Procédé selon l'une quelconque des revendications précédentes tel que la polymérisation 2) est réalisée en présence de $Si(OEt)_4$, $Si(OMe)_4$ ou $Si(O^iPr)_4$ par évaporation du solvant ou l'addition d'un catalyseur nucléophile et d'eau.

6.  Procédé de préparation d'un filtre pour capteur comprenant les étapes de :

1) préparation du nanomatériau hybride selon l'une quelconque des revendications 1 à 5 ;
2) dispersion dudit nanomatériau hybride dans un solvant organique ; et
3) dépôt de la suspension sur la surface de la couche sensible d'un capteur.

**Patentansprüche**

1. Verfahren zur Herstellung eines hybriden Nanomaterials, das metallische, gegebenenfalls oxidierte Nanopartikel enthält, umfassend:

   1) Bilden von Nanopartikeln des Metalls aus einem Precursor des Metalls in Anwesenheit eines Liganden (L), ausgewählt aus $(PhCH_2)_2N(CH_2)_{11}O(CH_2)_3Si(OEt)_3$ oder $H_2N(CH)_{11}O(CH_2)_3Si(OEt)_3$, bei Wasserstoffdruck;
   2) Polymerisieren des Liganden in einem Feststoff und
   3) Kalzinieren des erhaltenen Materials.

2. Verfahren nach Anspruch 1 derart, dass das Polymerisieren von einem Wasch- oder Trockenschritt vor dem Schritt des Kalzinierens gefolgt wird.

3. Verfahren nach Anspruch 1 oder 2 derart, dass das Metall ausgewählt ist aus Pd, Pt, Ru, Rh, Co, Ni, Fe, Au, Cu.

4. Verfahren nach einem beliebigen der vorhergehenden Ansprüche derart, dass der Metallprecursor Ru(COD)(COT) oder COD=1,5-cyclooctadien und COT=1,3,5-cyclooctatrien ist.

5. Verfahren nach einem beliebigen der vorhergehenden Ansprüche derart, dass das Polymerisieren in Anwesenheit von $Si(OEt)_4$, $Si(OMe)_4$ oder $Si(O^iPr)_4$ durch Verdampfen des Lösungsmittel oder Hinzufügen eines nukleophilen Katalysators und Wasser durchgeführt wird.

6. Verfahren zur Herstellung eines Filters für einen Sensor, die Schritte umfassend:

   1) Herstellen eines hybriden Nanomaterials nach einem beliebigen der Ansprüche 1 bis 5;
   2) Dispergieren des hybriden Nanomaterials in einem organischen Lösungsmittel; und
   3) Aufbringen der Suspension auf die Fläche der sensitiven Schicht des Sensors.

**Claims**

1. Method of preparing a hybrid nanomaterial containing possibly oxidized metal nanoparticles, comprising:

   1) the formation of nanoparticles of said metal from a precursor of said metal, in the presence of a ligand (L) selected from among $(PhCH_2)_2N(CH_2)_{11}O(CH_2)_3Si(OEt)_3$ or $H_2N(CH_2)_{11}O(CH_2)_3Si(OEt)_3$, under hydrogen pressure;
   2) the polymerisation of the ligand (L) within a solid material, and
   3) the calcination of the material thus obtained.

2. Method according to claim 1, wherein the polymerisation is followed by a step of washing or drying before the calcination step.

3. Method according to claim 1 or 2, wherein the metal is chosen from among Pd, Pt, Ru, Rh, Co, Ni, Fe, Au, Cu.

4. Method according to any one of the preceding claims, wherein the metal precursor is Ru(COD)(COT) or COD=1,5-cyclooctadiene and COT=1,3,5-cyclooctatriene.

5. Method according to any one of the preceding claims, wherein the polymerisation 2) is performed in the presence of $Si(OEt)_4$, $Si(OMe)_4$ or $Si(O^iPr)_4$ by evaporation of the solvent or the addition of a nucleophilic catalyst and water.

6. Method of preparing a sensor filter comprising the steps of:

   1) preparing the hybrid nanomaterial according to any one of claims 1 to 5;

2) dispersing said hybrid nanomaterial in an organic solvent; and
3) depositing the suspension on the surface of the sensitive layer of a sensor.

**Fig. 1**

Dépôt d'une suspension
du nanomatériau hybride par
micro- injecteur

**Fig. 2**

**sans filtre**

**avec filtre**

**Fig. 3**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **SCHWEIZER-BERBERICH et al.** *Sens. Actuators,* 2000, vol. 66, 34
- **ARBIOL et al.** *Applied Phys. Lett.,* 2002, vol. 81, 3449
- **LEE et al.** *Microporous and mesoporous materials,* 2004, vol. 70 (1-3), 71-80
- **CARPENTER et al.** *Microporous and mesoporous materials,* 2005, vol. 79 (1-3), 185-194
- **HUREA et al.** *Microporous and mesoporous materials,* 2005, vol. 79 (1-3), 185-194